# EUROPEAN PATENT SPECIFICATION

(11) **EP 2 653 137 B1**
(45) Date of publication and mention of the grant of the patent: **24.02.2016**
(21) Application number: 13164114.4
(22) Date of filing: 17.04.2013
(51) Int. Cl.: A61F 2/58, A61F 2/68

(54) **Self-contained multifunctional hand prosthesis**
Kompakte multifunktionelle Handprothese
Prothèse de main multifonctionnel et autonome

(30) Priority: 20.04.2012 IT PI20120049
(43) Date of publication of application: 23.10.2013
(73) Proprietor: Prensilia S.r.l., 56037 Peccioli, Pisa (IT)
(72) Inventor: Controzzi, Marco, 56124 PISA (IT); Clemente, Francesco, 03049 Sant'Elia Fiumerapido, Frosinone (IT); Cipriani, Christian, 55100 Lucca - San Concordio, Lucca (IT); Carrozza, Maria Chiara, 56123 Pisa (IT)
(74) Representative: Manzella & Associati

(56) References cited:
- JP-A- 2001 277 175
- US-A- 2 549 074
- US-A- 3 258 784
- US-A1- 2008 262 636

## Description

### TECHNICAL BACKGROUND OF THE INVENTION

The present invention relates to a hand prosthesis of the articulated type operated by motors. Such prosthesis is configured to reproduce the functions and dimensions of a hand, for example a human hand, in particular to replace a biological hand in its absence.

### DESCRIPTION OF THE PRIOR ART

Several attempts to make hand prostheses, especially human hand prostheses, to replace a biological hand in its absence, are known. People who have suffered the amputation of a hand in fact find themselves deprived of extremely important functions, essential for living self-sufficiently.

Such prostheses have the requirement to be as aesthetically similar to a biological hand as possible so as to make the user feel at ease and to prevent that in the presence of other peoples the difference is immediately noted.

To such purpose, all the mechanical and electronic components must therefore be integrated in the space typical of a normal hand both for aesthetic reasons and so as to permit the user to move the arm naturally. For the same reasons the need is felt to minimise the number of components, in particular the motors contained inside the prosthesis.

Among the attempts made to satisfy such requirements within the scope of the prior art, is the document US2008/0262636, which is considered to represent the closest prior art.

Such document tackles the problem of moving several fingers by means of a single drive input and comprises a rotating element positioned in the palm of the hand, to which an actuation rod for each finger to be moved is connected, each rod having a first end rotatably connected to the rotating element and an opposite second end rotatably connected to a phalanx of the corresponding finger in a point distant from the hinging point of the finger, so that when the rotating element is rotated by a predetermined angle in one direction, it pulls all the rods simultaneously and thus closes all the fingers simultaneously, while it pushes all the rods simultaneously by rotating in the opposite direction and thus opens all the fingers simultaneously.

The aforementioned patent document, while providing an actuation device of a small size and thus suitable for being integrated in the palm of the hand, has the considerable disadvantage of not permitting in any way the movement of the individual fingers to be separated.

Such known device therefore has the disadvantage of permitting the simultaneous closing and opening of all the fingers together, not individually.

In particular such prior device does not allow the flexion of the index finger when the flexion of the thumb finger is blocked, such as, for example, it would be required for allowing the prosthesis to press the trigger of a device with the index finger when the device is gripped by said prosthesis, in fact the flexion of the thumb finger might be blocked by the gripping of the tool itself.

In this situation the technical purpose at the basis of the present invention is to provide an articulated prosthesis of a hand which minimises the number of actuators, such as electric motors needed to independently move several degrees of freedom and which, at the same time, allows the thumb finger to perform various types of gripping.

These and other purposes are achieved by an articulated hand prosthesis according to the present invention, having a metacarpus which extends substantially along a metacarpal plane, and at least one thumb finger having a proximal phalanx rotatably connected to the metacarpus and an index finger having a proximal phalanx rotatably connected to the metacarpus, comprising flexion means of the index finger suitable for making the index finger rotate around a flexion axis substantially parallel to the metacarpal plane.

In particular, the prosthesis comprises adduction means of the thumb finger suitable for making the thumb finger rotate around an adduction axis substantially parallel to the metacarpal plane, and inclined with respect to the flexion axis.

The prosthesis, comprises in addition at least one actuator for example an electric motor integrated in the prosthesis.

Alternatively, the prosthesis comprises a housing for an actuator, suitable for integrating said actuator inside the metacarpus.
the prosthesis further comprises motion transmission means of the movement between the actuator and said flexion and adduction means, said motion transmission means being of the mechanical type.

The motion transmission means are configured so as to control in a substantially selective manner said flexion means and said adduction means.
In particular the motion transmission means are configured to block the adduction means of the thumb finger and simultaneously move the flexion means of the index finger to open/close during at least one movement or portion of movement of the actuator.
The features and advantages of the invention will be evident from the detailed description below of a preferred embodiment of the invention, with reference to the appended drawings, wherein:

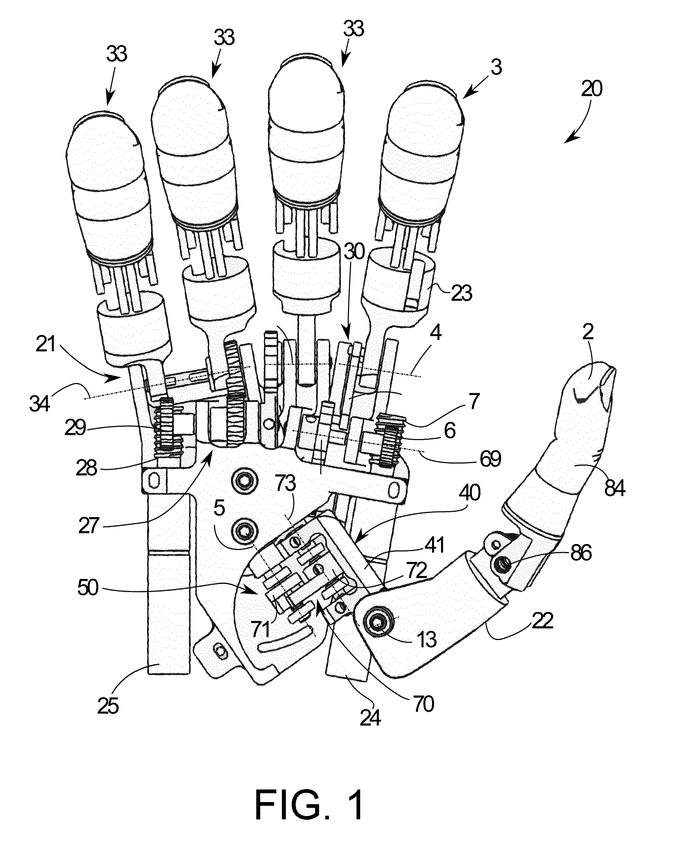

- figure 1 shows an example of a hand prosthesis according to the invention seen from the palm and having an open hand posture;
- figures 2 and 3 shows the prosthesis seen from the palm and a side view during a particular gripping posture called side grip;

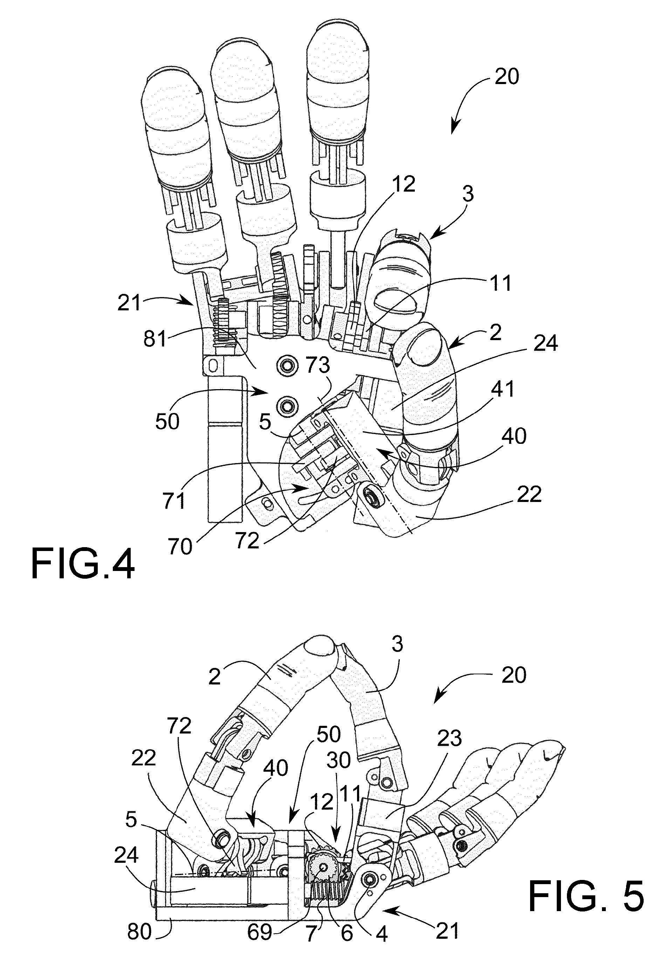

- figures 4 and 5 show the prosthesis seen from the palm and in a side view during a precision gripping using the thumb finger and index finger;
- f i gu r e 6 is a cross-section view along a plane parallel to the metacarpal plane and passing through the motor axis in the position of maximum adduction of the thumb finger and minimum flexion of the index finger;

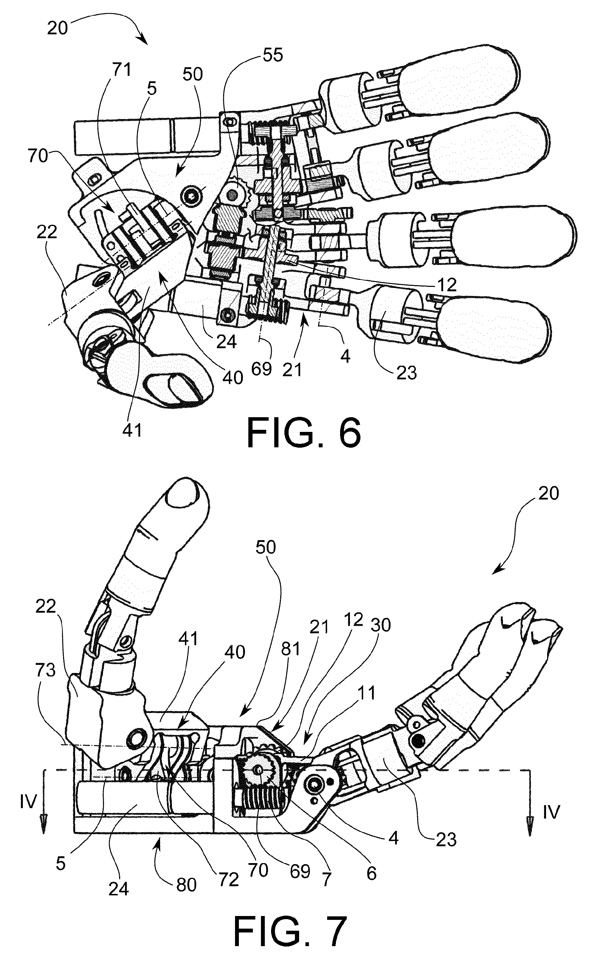

- figure 7 shows the prosthesis in the same posture as in figure 6 from a side view;

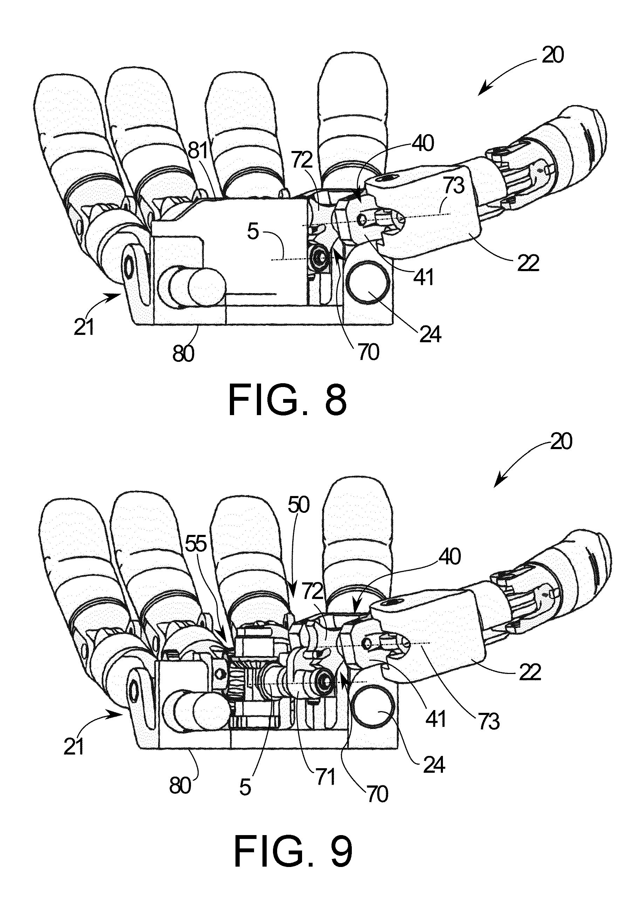

- figures 8 and 9 show the prosthesis seen from the wrist with the thumb finger in the position of minimum adduction and the index finger in the position of minimum flexion, respectively with the palm element fitted and removed to show the inner components;

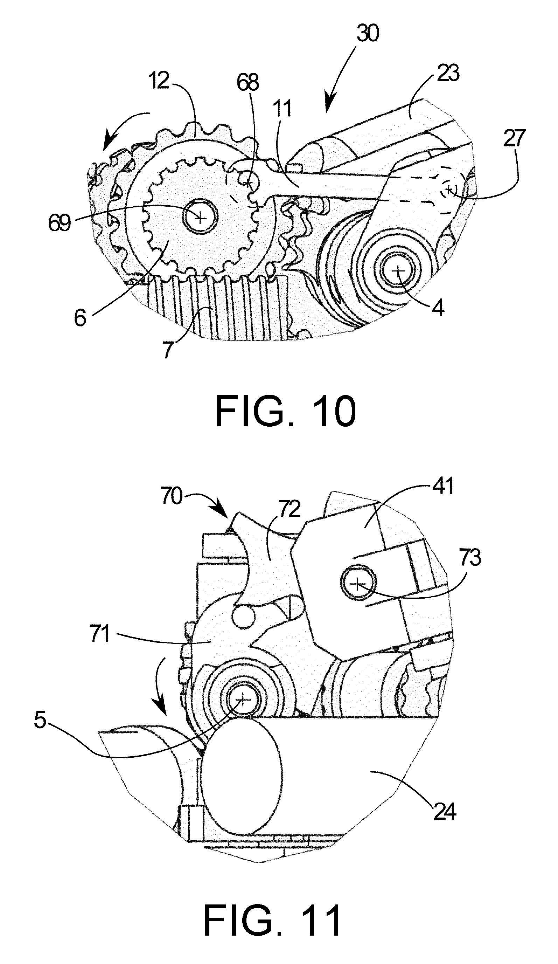

- figure 10 shows a detail of the flexion means;
- figure 11 shows a detail of the adduction means of the thumb finger;
- figures 12 and 13 show the flexion and adduction means passing from a first to a second angular position of the main rotating element;
- figure 14 shows the hand prosthesis passing from a first to a second angular position of the main rotating element as in figures 12 and 13;
- figures 15 and 16 show the flexion and adduction means passing from a second to a third angular position of the main rotating element;
- figure 17 shows the hand prosthesis passing from a second to a third angular position of the main rotating element as in figures 15 and 16;
- figures 18 and 19 show the flexion and adduction means passing from a third to a fourth angular position of the main rotating element;
- figure 20 shows the hand prosthesis passing from a third to a fourth angular position of the main rotating element as in figures 18 and 19.

With reference to the appended drawings, the articulated hand prosthesis according to the invention is generally indicated by reference number 20.

Such prosthesis is preferably suitable for reproducing a human hand in its dimensions and movements, but does not exclude reproducing a hand of an animal having a metacarpus and fingers suitable for flexion and adduction movements.

"Metacarpus" is defined as a portion of the hand from which the fingers extend, defined underneath by the palm and above by the back of the hand. The metacarpus substantially extends along a plane. "Metacarpal plane" is taken to mean a substantially median plane in relation to the metacarpus.

"Proximal phalanx" of the fingers means the phalanx of each finger directly connected to the metacarpus, while "distal phalanx" is that furthest away from the metacarpus.

"Flexion" means the rotation of the proximal phalanx along a plane substantially orthogonal to the metacarpal plane and around an axis parallel to the metacarpal plane, while "flexion axis" refers to said rotation axis.

"Adduction of the thumb finger" means the rotation of the proximal phalanx of the thumb finger along a plane substantially orthogonal to the metacarpal plane, around a rotation axis substantially parallel to the metacarpal plane but inclined in relation to the flexion axis. Such rotation axis of the thumb finger is called the thumb finger adduction axis.

The metacarpus 21 comprises a back 80 and a palm 81, substantially parallel to the back. In particular but not necessarily, these may both be substantially flat, or curved so as to replicate curves typical of a biological hand. The palm may be defined as a removable closure element for allowing the access to a space enclosed between the palm and back, called the "carpal space", suitable for containing all the mechanical, transmission and any electronic components so as to make a compact and integrated prosthesis. The back comprises a hinge element in the part facing the fingers around the flexion axes 4, 34, 35 of the fingers 3, 33. The thumb finger too is hinged to the metacarpus in the inner part of the back.

The fingers 3, 33 each comprise three articulated phalanxes. Each phalanx is rotatably connected to the next around respective axes substantially parallel to the flexion axes 4, 34, 35.

The thumb finger 2 comprises a support element 41 rotatably connected to the inner part of the back around the adduction axis 73, a proximal phalanx 22 rotatably connected to the support element 41 around a transversal axis 13 in relation to the adduction axis, a distal phalanx 84 hinged to the proximal phalanx around an axis 86 substantially parallel to the axis 13.

According to a general embodiment of the invention, the articulated hand prosthesis 20 comprises flexion means 30 of the index finger 3 suitable for making the index finger 3 rotate around a flexion axis 4 substantially parallel to the metacarpal plane M-M.

The prosthesis further comprises adduction means 40 of the thumb finger 2 suitable for making the thumb finger 2 rotate around an adduction axis 73 substantially parallel to the metacarpal plane M-M, and inclined in relation to the flexion axis 4. In one example which is not in accordance with the present invention, the prosthesis comprises a housing for an actuator 24, for example for an electric motor, said housing being suitable to receive said actuator inside the prosthesis, for example inside the metacarpus 21.

In other words, the prosthesis comprises an actuator housing 24, configured to receive within it an actuator 24. For example, the prosthesis does not comprise the motor but is predisposed to receive it.

According to the invention, the prosthesis, comprises at least one electric motor 24 integrated in the prosthesis, for example in the metacarpus.

According to one embodiment, the aforesaid electric motor is a single electric rotary motor.

Such electric motor, in particular, has a rotating motor shaft suitable for being connected to motion transmission means of the movement from said motor to said flexion and adduction means.

According to one embodiment, the prosthesis comprises an electric motor 24 integrated in the prosthesis 20 and motion transmission means 50 of the movement from the electric motor 24 to the flexion 30 and adduction 40 means, said motion transmission means 50 being of the mechanical type wherein such motion transmission means 50 are configured so as to control in a substantially selective manner said flexion means 30 and said adduction means 40.

In other words, the prosthesis according to the invention, allows the flexion movement of the index finger to be separated from the adduction movement of the thumb finger, even though both movements are operated by means of the same electric motor. Depending on the angular position of the shaft of the electric motor the index finger or the thumb finger is separately controlled. Starting from the end position of the wheel 12 in which the index finger is flexed in a closed position and the thumb finger is adducted in a minimum position, in a first angular range, the flexion for the opening of the index finger only is operated, in a subsequent angular range the adduction of the thumb finger only is performed with an extremely slight flexion of the index finger which leaves the cavity of the palm in any case open and free, in a third angular range the flexion for the closing of the index finger only is performed. The transmission is purely mechanical and avoids having to introduce segments of transmission operated by cables or separate electro-mechanical actuators.

According to one embodiment, in fact, the motion transmission means 50 of the articulated prosthesis 20 are configured to transform a continuous rotary motion of a main rotating element 12 driven by the motor 24, into an intermittent or alternating motion, of the adduction means 40 and into an oscillatory motion of the flexion means 30.

The prosthesis 20 thus comprises the main rotating element 12.

Moreover, in such articulated prosthesis 20 the motion transmission means 50 is configured so that a first angular movement 60 of the main rotating element 12 between a first angular position 61 and a second angular position 62, corresponds to an evident flexion rotation 66 of the proximal phalanx 23 of the index finger 3 and a blocked position of the adduction rotation of the adduction means 40 of the thumb finger 2.

In other words, the motion transmission means 50 are configured so that, in correspondence with at least one movement of the electric motor 24, between a first position and a second position, the flexion means 30 of the index finger are operated to open or to close, and simultaneously the adduction means 40 of the thumb finger are kept blocked.

Consequently, the motion transmission means 50 are configured to keep the adduction means 40 of the thumb finger blocked and simultaneously operate the flexion means 30 of the index finger to open/close, in correspondence with at least one movement of the electric motor 24, between a first position and a second position.

Again, in other words, the motion transmission means 50 are configured to block the proximal phalanx 22 of the thumb finger 2 and simultaneously move the proximal phalanx 23 of the index finger 3 to open/close, in correspondence with at least one movement of the electric motor 24, between a first position and a second position.

In yet other words, the motion transmission means 50 are configured so that at least one movement, in particular of the electric motor, corresponds to an evident flexion rotation 66 of the proximal phalanx 23 of the index finger 3 and a blocked position of the rotation of the adduction means 40 of the thumb finger 2.

Preferably, the first angular position and second angular position are respectively comprised between an angle of 150° and an angle of 20° in relation to an axis parallel to the metacarpal plane M-M and having the anti-clockwise direction as the positive direction of rotation.

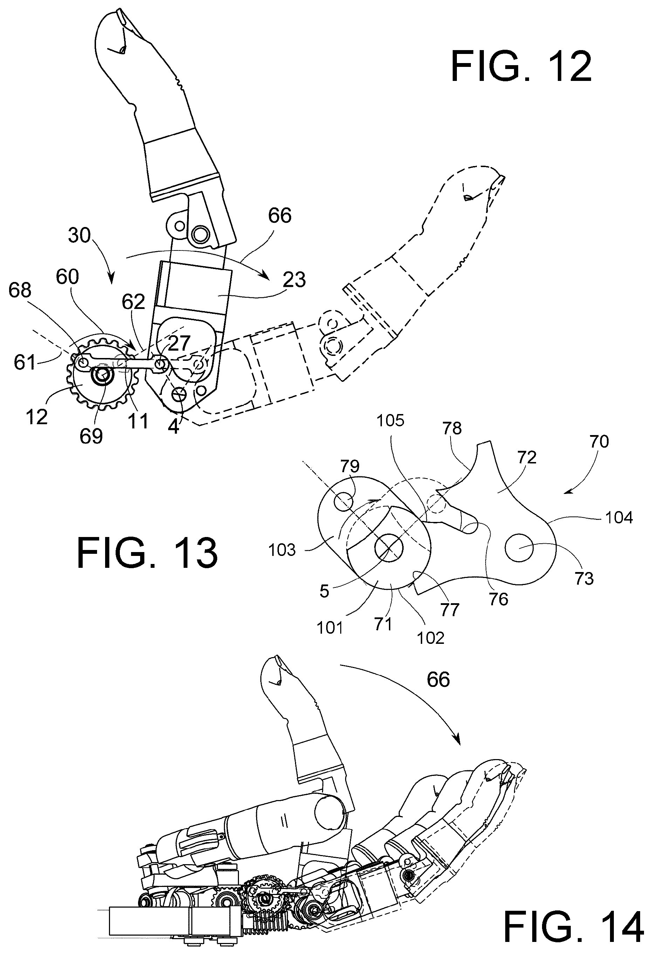

During such movement as shown in figures 12, 13 and 14, the proximal phalanx 23 of the index finger moves from a closed position to an open position, while the adduction means of the thumb finger remain blocked in a minimum adduction position, that is with the proximal phalanx 22 of the thumb finger blocked in the point of minimum rotation towards the inside of the palm.

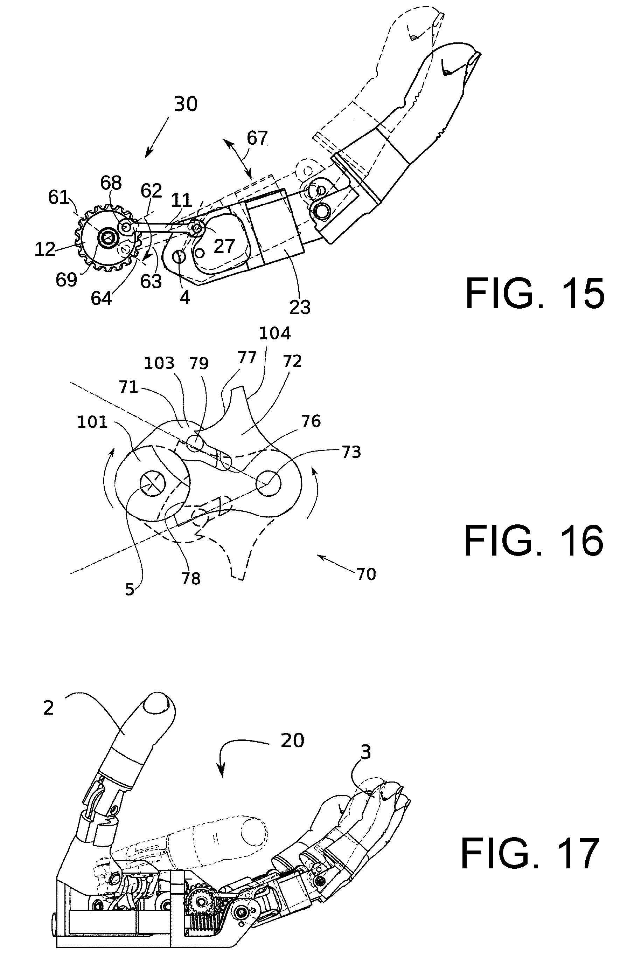

Moreover, as shown in figures 15 to 17, the motion transmission means 50 may be configured so that a second angular movement 63 of the main rotating element 12 between a second angular position 62 and a third angular position 64, corresponds to a position of substantial absence of rotation of the proximal phalanx of the index finger 23 and an evident rotation of the adduction means 40 of the thumb finger 2.

Preferably, the second angular position 62 and the third angular position 63 are respectively comprised between an angle of 20° and an angle of -20° in relation to an axis parallel to the metacarpal plane M-M and having the anti-clockwise direction as the positive direction of rotation. During such movement, the proximal phalanx of the index finger 23 remains in a position of minimal flexion, that is to say substantially aligned with the metacarpus, in an open position, while conversely, the proximal phalanx of the thumb finger 22 performs an adduction movement, that is to say is rotated so as to close towards the inside of the palm, tending to align with the metacarpus. In this step, the main element 12 rotates between the second angular position 62 and the third angular position 64, this rotation 63 induces a very small angular rotation 67 of the proximal phalanx of the index finger 23 around the flexion axis 4.

In one embodiment, by means of simple trigonometric formulas it may be affirmed that the angle of rotation 67 of the proximal phalanx 23 of the index finger is equal to the arcsine of the cosine of half the angular rotation 63 of the rotating element 12, multiplied by the ratio of the distance between a first rotating point of constraint 68 and the rotation axis 69, and the distance between a second rotating point of constraint 27 and the flexion axis 4, where the first point of constraint 68 is between the main rotating element 12 and one end of the connecting rod 11, and the second point of constraint 27 is between a second end of the connecting rod 11 and the proximal phalanx 23 of the index finger.

Consequently, since between a second angular position 62 and a third angular position 63 of the rotating element 12, the angle of rotation 67 of the proximal phalanx 23 is an extremely small value, it may be affirmed that such phalanx 23 in such range is substantially stationary.

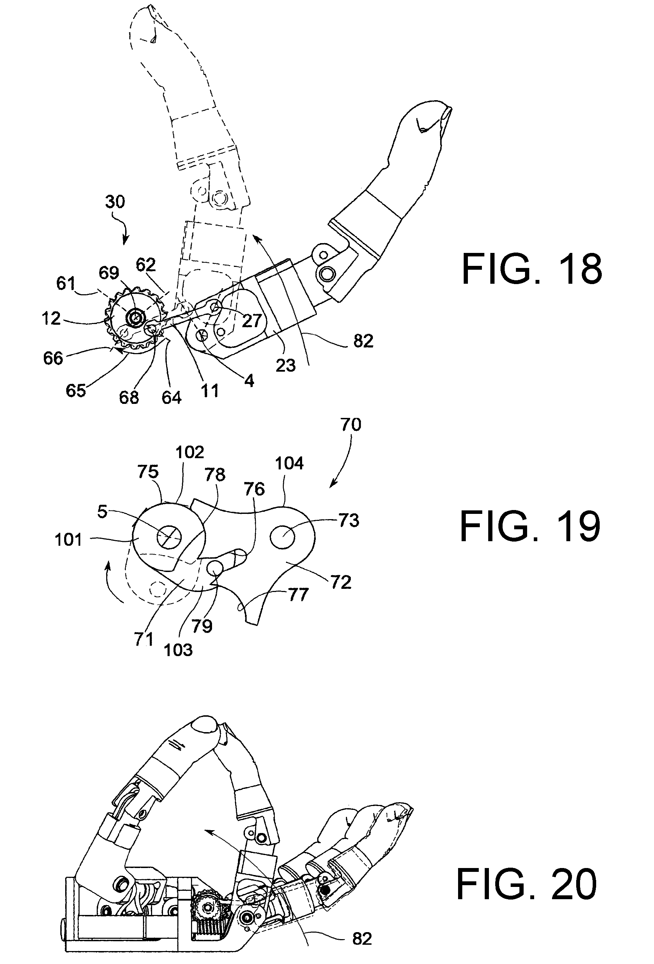

As shown in figures 18-20, the articulated prosthesis 20, may also be configured so that a third angular movement 65 of the main rotating element 12 between the third angular position 64 and a fourth angular position 66 corresponds to an evident flexion rotation 82 of the proximal phalanx 23 of the index finger 3 and a blocked position of the adduction rotation of the adduction means 40 of the thumb finger 2.

Preferably, the third angular position and the fourth angular position are respectively defined by an angle of -20° and an angle of -150° in relation to an axis parallel to the metacarpal plane M-M and having the anti-clockwise direction as the positive direction of rotation.

By controlling the rotation of the shaft of the motor 24 it is possible to control the rotation of the main rotating element 12, and consequently simultaneously regulate the positions of the index finger and of the thumb finger.

According to one embodiment, the adduction means 40 of the thumb finger 2 comprise a rigid support 41 rotatably connected to the proximal phalanx 22 of the thumb finger through the axis 13, said support 41 being rotatably connected to the metacarpus 21 around the adduction axis 73.

The support 41 is preferably, but not necessarily, substantially U-shaped.
the rotation axis 5 is kinematically connected to the main rotating element 12. Said rotation axis 5 is rotatably connected to the motor element 71 of a Malta or Geneva cross element 70. The driven element 72 of the mechanism 70 is connected to the rigid support 41 which is rotatably connected about the adduction axis 73.

In other words, according to one embodiment, the motion transmission means 50 comprise a Malta or Geneva cross mechanism 70.

According to one embodiment, the aforesaid Malta or Geneva cross mechanism 70, comprises a drive wheel 71 having a rotation axis 5, or motor axis, and a driven wheel 72 having a rotation axis coinciding with or parallel to the adduction axis 73.

The drive wheel 71 of the mechanism is kinematically connected to said main rotating element 12 and the driven wheel 72 of the mechanism is integral with the adduction means 40 of the thumb finger 2.

In other words, the rotation of the drive wheel 71 is kinematically connected to the rotation of the main rotating element 12, the rotation of which is connected to the movement of the proximal phalanx 23 of the index finger, while the rotation of the driven wheel 72 is connected to the adduction rotation of the proximal phalanx 22 of the thumb finger 2.

The drive wheel 71 comprises a cylindrical portion 101 coaxial to the rotation axis of the wheel 71 and laterally defined by a cylindrical lateral surface 102.

The drive wheel comprises, in addition, a portion 103 radially projecting from an end base of said cylindrical portion 101, said radially projecting portion 103 comprising a follower pin 79 substantially parallel to the rotation axis 5, at a predefined distance from said rotation axis 5. In other words, the drive wheel 71 is configured to rotate around the rotation axis 5, so that the follower pin 79 travels over a circumference around the rotation axis 5.

The driven wheel 72 has an outer lateral surface 104 comprising a first concave surface portion 77 and a second concave surface 78 separated from each other by a radial slot 76, radially open and configured to slidingly receive the pin 79, wherein said first concave surface 77 and said second concave surface 78 are portions having a cylindrical surface, the same as each other and symmetrically arranged in relation to a radial plane passing through the rotation axis 73 and passing centrally to the radial slot 76, wherein said first surface and second cylindrical surface are complementary to the cylindrical surface 102 of the drive wheel 71.

The distance between the rotation axis 73 of the driven wheel 72 and the rotation axis 5 of the drive wheel is chosen so that the concave surface 77 and the concave surface 78 can come into contact with the lateral cylindrical surface 102 of the drive wheel 71.

According to one embodiment, the radial slot 76 has a mouth 105 having input surfaces which permit or facilitate the entrance and exit of the pin 79 into or out of the slot 76 during the rotation of the drive wheel 71.

According to one embodiment, the concave surfaces 77 and 78, the slot 76 and even the input surfaces of the mouth 105, are geometrically generated by a straight line moving parallel to the rotation axis 73 of the driven wheel.

In figure 13, the drive wheel is shown in an angular position in which the pin 79 is outside the slot 76 and the lateral surface of the drive wheel touches the first concave surface 77. In this configuration the drive wheel 71 may rotate in a clockwise direction while the driven wheel is blocked, and no movement of the driven wheel is permitted. Consequently, in the configuration in figure 13, the proximal phalanx 23 of the index finger rotates while the proximal phalanx 22 of the thumb finger 2 is kept blocked. This configuration corresponds to the aforesaid first angular range in which the flexion upon opening of the index finger only is performed, also shown in figures 12 and 14.

In figure 16 the drive wheel 71 continues to rotate in a clockwise direction and is shown at the entrance of the pin 79 in the slot 76, at one end of the aforesaid first angular range. In this situation the aforesaid second angular range begins, in which the proximal phalanx of the index finger is substantially stationary or moves slowly, while the proximal phalanx of the thumb finger is made to rotate.

Within the second angular range shown in figure 16, in fact, the pin 79 of the drive wheel 71 makes the driven wheel 72 rotate thus moving the proximal phalanx of the thumb finger.

According to one embodiment, the aforesaid second angular range is chosen around an angular position corresponding to a dead centre or point of singularity of the movement of the flexion means of the index finger, so that in such range the proximal phalanx of the index finger remains substantially stationary.

For example, said dead centre or point of singularity occurs in correspondence with the alignment of :
- a first rotating point of constraint 68 between the main rotating element 12 and one end of a connecting rod element 11;
- a second rotating point of constraint 27 between a second end of the connecting rod element 11 and the proximal phalanx 23 of the index finger;
- the rotation axis or motor axis 69 of the main rotating element 12.

In figure 19, the drive wheel 71 is shown leaving the second angular range, continuing to rotate in a clockwise direction and entering the aforesaid third angular range. In the position shown in the figure, the following pin 79 is coming out of the radial slot 76 and the second concave surface 78 is touching the lateral surface 102 of the drive wheel 71. In this position the driven wheel 72 is blocked while the drive wheel 71 can continue to rotate. The thumb finger is thus blocked in an angular position, while the index finger rotates to open in the direction of the arrow 66.

According to one embodiment, the adduction means 40 of the thumb finger 2 comprise a rigid support 41 substantially U-shaped and connected to the proximal phalanx 22 of the thumb finger 2.

The support 41 is rotatably connected to the metacarpus 21 around the adduction axis 73, said rotation axis 73 being rotatably connected to said driven element 72 of the Malta or Geneva cross element 70, said drive wheel 71 of the mechanism 70 being rotatably connected to a motor axis 5, substantially parallel to the adduction axis 73.

According to one embodiment, the flexion means 30 comprise the main element 12 rotating around a motor axis 69, substantially parallel to the flexion axis 4, and a connecting rod element 11 having a first end hinged to said main rotating element 12 in a rotating point of constraint 68 external to the motor axis 69, and a second end hinged to the proximal phalanx 23 of the index finger in a rotating point of constraint 27 external to the flexion axis 4.

The flexion means can thus be schematised as an articulated quadrilateral, in which the elements rotatably connected at fixed points are formed by the main rotating element 12 which constitutes the crankshaft of said articulated quadrilateral, and by the proximal phalanx 23 of the index finger which constitutes the rocker arm, while the element rotatably connected to both is the connecting rod element 11. According to one embodiment, the connecting rod element may consist of materials with varying elastic characteristics in traction, compression and flexion, such as for example a spring or a cable or a combination thereof, and be suitably shaped to reproduce such properties.

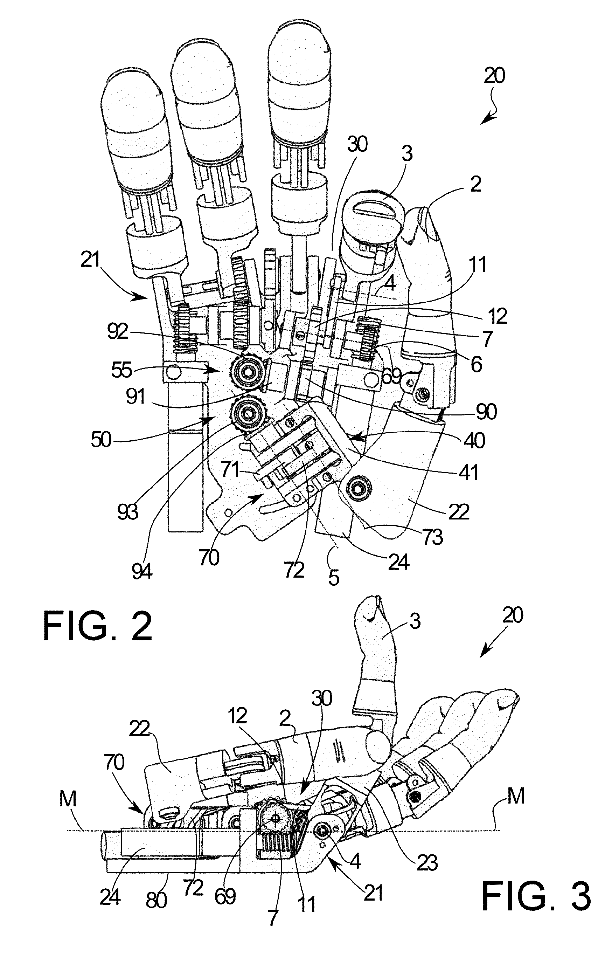

The motion transmission means 50 comprise a transmission line of the movement 55 between the main rotating element 12 and the motor element 71 of the mechanism 70. For example as shown in figure 2, the main rotating element 12 meshes in a driven wheel 90 fitted coaxially and integral with an end bevel wheel 91, which meshes on another bevel wheel 92, kinematically connected to a further bevel wheel 93 which transmits the movement to another bevel wheel 94 integral and coaxial with the motor element 71 of the mechanism 70.

In one embodiment, the main rotating element 12 and the motor element 71 are kinematically coupled by means of sectors of cogwheels which mesh each other, or cog wheels which mesh each other.

In one embodiment, the electric motor 24 is an electric motor of the rotary type having a worm 7 fitted on its shaft, said shaft 7 meshing with an input cogwheel of the movement 6, said input cogwheel 6 being coaxial and integral with said main rotating element 12. The motor 24 for example, but not necessarily is of the electric type with brushes or brushless.

The articulated prosthesis 20 may comprise, as well as the thumb finger 2 and the index finger 3, the remaining three fingers 33 of the hand hinged to respective flexion axes 34, 35, operated by a second electric rotary motor 25 integrated in the prosthesis 20, said motor 25 comprising a worm 28 fitted on its shaft, coupled to a further cogged wheel 29 connected to a second transmission line 27 which operates the flexion motion of the further three fingers 33.

The flexion motion of the distal phalanx 84 in relation to the proximal phalanx 22 of the thumb finger 2 may be controlled by means of a further electric motor (not shown in the drawings) fitted inside such proximal 22 or distal 84 phalanx of the thumb finger.

The flexion motion of the distal phalanx in relation to the proximal phalanx 23 of the index finger 3 may be operated by a further electric motor (not shown in the drawings) inserted inside the proximal or distal phalanx.

The flexion motion of the distal phalanx of the remaining fingers 33, may be operated by further respective electric motors (not shown in the drawings).

The flexion motion of the proximal phalanx 22 of the thumb finger 2 may be controlled by means of the main rotating element 12 operated by the motor 24 by means of a further movement transmission line (not shown in the drawings).

The invention described brings the following important advantages.

The provision of controlling in a substantially selective manner the flexion means of the index finger or the adduction means of the thumb finger by means of the same one electric motor, makes it possible to minimise the number of electric motors needed to control different movements of different fingers.

The fact of substantially selectively controlling the flexion means of the index finger or the adduction means of the thumb finger by means of the same electric motor, also makes it possible to use almost all the torque supplied by the main rotating element operated by the motor, selectively for the flexion movement of the index finger or the adduction means of the thumb finger when the index finger is in the open position and thus when it may be supposed that gripping loads are not weighing on such finger. Said substantial selective selection of the movements brings considerable advantages in terms of efficiency and gripping performance.

The provision of providing motion transmission means which make it possible to transform the continuous motion of a main rotating element driven by the motor, into intermittent motion, for example alternate or discontinuous of the adduction means, makes it possible to associate to predefined separate angles of rotation of the main rotating element, different postures and functions of the index finger and thumb finger.

This entails an advantage in that it is sufficient to position the main rotating element at a predefined angle to induce a combined flexion/extension of the index finger and /or adduction/abduction movement of the thumb finger, and permit functions associated with the single fingers, such as pressing buttons on a keypad, or gripping functions. Said gripping functions can be achieved by the subsequent movement produced by the motor installed in the thumb finger, which depending on the adduction position, makes it possible to perform palm gripping, namely in which the thumb finger closes towards the palm of the hand, or lateral gripping, namely when the thumb finger closes towards the outer lateral surface of the index finger.

The prosthesis according to the invention has the added advantage of combining the contrasting requirements of making the prosthesis as similar as possible in appearance to the human hand and at the same time of containing inside it all the mechanics ad actuators needed for full functionality thereof, naturally reproducing all the movements of the human hand.

The above description of a specific embodiment is able to show the invention from a conceptual point of view, so that others, using the prior art, may modify and/or adapt such embodiment in various applications without further research and without straying from the inventive concept, and consequently such adaptations and modifications shall be considered as equivalent to the embodiment described. The means and materials for performing the various functions described may be varied while remaining within the scope of the invention. It is understood that the expressions or terms used have purely descriptive purposes and hence not limiting.

## Claims

1. A n articulated hand prosthesis (20) having a metacarpus (21) which extends substantially along a metacarpal plane (M-M), and at least one thumb finger (2) having a proximal phalanx (22) rotatably connected to the metacarpus (21) and an index finger (3) having a proximal phalanx (23) rotatably connected to the metacarpus (21), comprising:
- flexion means (30) of the index finger (3) suitable for making the index finger (3) rotate around a flexion axis (4) substantially parallel to the metacarpal plane (M-M) ;
- adduction means (40) of the thumb finger (2) suitable for making the thumb finger (2) rotate around an adduction axis (73) substantially parallel to the metacarpal plane (M-M), and inclined with respect to the flexion axis (4) of the index finger;
- an electric motor (24) integrated in the prosthesis (20) ;
- motion transmission means (50) between the electric motor (24) and the flexion means(30) and adduction means (40), said motion transmission means (50) being of the mechanical means;
- a main rotating element (12) driven by the electric motor (24);
- said motion transmission means (50) are arranged in order to drive in a substantially selective manner said flexion means (30) and said adduction means (40);
**characterized in that**:
- said motion transmission means (50) are configured so that a first angular movement (60) of the main rotating element (12) between a first angular position (61) and a second angular position (62), causes a flexion rotation (66) of the proximal phalanx (23) of the index finger (3) between a closed position and an open position, wherein the adduction means (40) of the thumb finger (2) remain blocked during said first angular movement (60) of the main rotating element (12) between the first angular position (61) and the second angular position (62)

2. The articulated prosthesis according to claim 1, wherein the motion transmission means (50) are arranged in order to keep the adduction means (40) of the thumb finger blocked and to simultaneously operate the flexion means (30) of the index finger to open/close, in correspondence with at least one movement provided by the electric motor (24) from a first position to a second position.

3. The articulated prosthesis according to claim 1 or 2, wherein said motion transmission means (50) are configured to transform a continuous rotary movement of the main rotating element (12) actuated by the motor (24), into an intermittent movement of the adduction means (40).

4. The articulated prosthesis according to claim 3, wherein said motion transmission means (50) are configured so that:
- said first angular movement (60) of the main rotating element (12) between the first angular position (61) and the second angular position (62), corresponds to an evident flexion rotation (66) of the proximal phalanx (23) of the index finger (3) and a blocking position of the rotation of the adduction means (40) of the thumb finger (2);
- a second angular movement (63) of the main rotating element (12) between the second angular position (62) and a third angular position (64), corresponds to a position of substantial absence of rotation of the proximal phalanx (23) of the index finger (3) and an evident rotation (87) of the adduction means (40) of the thumb finger (2);
- a third angular movement (65) of the main rotating element (12) between the third angular position (64) and a fourth angular position (66) corresponds to an evident flexion rotation (82) of the proximal phalanx (23) of the index finger (3) and a blocking position of the rotation of the adduction means (40) of the thumb finger (2).

5. The articulated prosthesis according to claim 4, wherein the motion transmission means (50) comprise a Malta or Geneva cross mechanism (70), wherein the drive wheel (71) of the mechanism is kinematically connected to said main rotating element (12) and, the driven wheel (72) of the mechanism is integral with the adduction means (40) of the thumb finger (2).

6. The articulated prosthesis according to claim 5, wherein the adduction means (40) of the thumb finger (2) comprise a rigid frame (41) substantially U-shaped and connected to the proximal phalanx (22) of the thumb finger (2), said support (41) being rotatably connected to the metacarpus (21) around the adduction axis (73), said adduction axis (73) being rotatably connected to said driven wheel (72) of the Malta or Geneva cross element (70), said drive wheel (71) of the mechanism (70) being rotatably connected to a motor axis (5) substantially parallel to the adduction axis (73).

7. The articulated hand prosthesis, according to claim 1, wherein said flexion means (30) comprise the main element (12) rotating around a motor axis (69), substantially parallel to the flexion axis (4), and a connecting rod element (11) having a first end hinged to said main rotating element (12) in a point (68) external to the motor axis (69), and a second end hinged to the proximal phalanx (23) of the index finger in a point (27) external to the flexion axis (4).

8. The articulated prosthesis, according to claims 3 and 7, wherein said connecting rod element (11) is comprised of elastic materials, springs or cables or a combination thereof.

9. The articulated prosthesis, according to claims 3 and 7, wherein the motion transmission means (50) comprise a transmission line of the movement (55) between the main rotating element (12) and the drive wheel (71) of the mechanism (70).

10. The articulated prosthesis, according to claims 3 and 7, wherein the main rotating element (12) and the drive wheel (71) are sectors of a cogwheel which mesh each other, or cogged wheels which mesh each other.

11. The articulated prosthesis according to any of the previous claims, wherein the electric motor (24)is of the rotary type having a worm (7) fitted on its shaft, said worm (7) meshing with an input cogwheel of the movement (6), said input cogwheel (6) being coaxial and integral with said main rotating element (12).

12. The articulated prosthesis (4) according to any of the previous claims, comprising, as well as the thumb finger (2) and the index finger (3), the remaining three fingers (33) of the hand hinged to respective flexion axes (34, 35), operated by a second electric rotary motor (25) integrated in the prosthesis (20), said second motor (25) comprising a worm (28) fitted on its shaft, coupled to a further cogwheel (29) connected to a second transmission line (27) which operates the flexion motion of the further three fingers (33) around said flexion axes (34, 35).

## Patentansprüche

1. Hand-Gelenkprothese (20), aufweisend eine Mittelhand (21), die sich im Wesentlichen entlang einer Mittelhandebene (M-M) erstreckt, und mindestens einen Daumen (2), aufweisend eine Grundphalanx (22), die drehbar mit der Mittelhand (21) verbunden ist, und einen Zeigefinger (3), aufweisend eine Grundphalanx (23), die drehbar mit der Mittelhand (21) verbunden ist, umfassend:
- mittel zum Beugen (30) des Zeigefingers (3), die ausgelegt sind, um den Zeigefinger (3) um eine Beugungsachse (4) zu drehen, die im Wesentlichen parallel zur Mittelhandebene (M-M) verläuft;
- mittel zur Adduktion (40) des Daumens (2), die ausgelegt sind, um den Daumen (2) um eine Adduktionsachse (73) zu drehen, die im Wesentlichen parallel zur Mittelhandebene (M-M) verläuft und zur Beugungsachse des Zeigefingers (4) geneigt ist;
- einen Elektromotor (24), der in die Prothese (20) integriert ist;
- mittel zur Übertragung der Bewegung (50) zwischen dem Elektromotor (24) und den Mitteln zum Beugen (30) und zur Adduktion (40), wobei diese Übertragungsmittel (50) mechanischer Art sind;
- ein rotierendes Hauptelement (12), das über den Elektromotor (24) betätigt wird;
- wobei die Übertragungsmittel (50) ausgebildet sind, um die Beugungsmittel (30) und die Adduktionsmittel (40) im Wesentlichen selektiv zu steuern;
**dadurch gekennzeichnet, dass**:
- die Übertragungsmittel (50) so ausgebildet sind, dass eine erste Winkelbewegung (60) des rotierenden Hauptelements (12) zwischen einer ersten Winkelposition (61) und einer zweiten Winkelposition (62) eine deutliche Beugedrehung (66) der Grundphalanx (23) des Zeigefingers (3) zwischen einer geschlossenen und einer offenen Position erzeugt, während die Adduktionsmittel (40) des Daumens (2) während der ersten Winkelbewegung(60) des rotierenden Hauptelements (12) zwischen der ersten Winkelposition (61) und der zweiten Winkelposition (62) blockiert bleiben.

2. Gelenkprothese nach Anspruch 1, wobei die Übertragungsmittel (50) ausgebildet sind, um die Adduktionsmittel (40) des Daumens blockiert zu halten und gleichzeitig das Öffnen/Schließen der Beugungsmittel (30) des Zeigefingers bei mindestens einer Bewegung des Elektromotors (24) von einer ersten Position bis zu einer zweiten Position zu steuern.

3. Gelenkprothese nach Anspruch 1 oder 2, wobei die Übertragungsmittel (50) ausgelegt sind, um eine kontinuierliche Drehbewegung eines rotierenden Hauptelements (12), die durch den Motor (24) ausgelöst wird, in eine intermittierende Bewegung der Adduktionsmittel (40) umzuwandeln.

4. Gelenkprothese nach Anspruch 3, wobei die Übertragungsmittel (50) so ausgebildet sind, dass:
- der ersten Winkelbewegung (60) des rotierenden Hauptelements (12) zwischen der ersten Winkelposition (61) und der zweiten Winkelposition (62) eine deutliche Beugedrehung (66) der Grundphalanx (23) des Zeigefingers (3) und eine Blockierposition der Drehung der Adduktionsmittel (40) des Daumens (2) entspricht;
- einer zweiten Winkelbewegung (63) des rotierenden Hauptelements (12) zwischen der zweiten Winkelposition
- und einer dritten Winkelposition (64) eine Position entspricht, in der im Wesentlichen keine Drehung der Grundphalanx (23) des Zeigefingers (3) erfolgt, sowie eine deutliche Drehung (87) der Adduktionsmittel (40) des Daumens (2);
- einer dritten Winkelbewegung (65) des rotierenden Hauptelements (12) zwischen der dritten Winkelposition (64) und einer vierten Winkelposition (66) eine deutliche Beugedrehung (68) der Grundphalanx (23) des Zeigefingers (3) und eine Blockierposition der Drehung der Adduktionsmittel (40) des Daumens (2) entspricht.

5. Gelenkprothese nach Anspruch 4, wobei die Übertragungsmittel (50) einen Malteserkreuz- oder Sternradmechanismus (70) umfassen, wobei das Antriebsrad (71) des Mechanismus kinematisch mit dem rotierenden Hauptelement (12) verbunden ist und das angetriebene Rad (72) des Mechanismus fest mit den Adduktionsmitteln (40) des Daumens (2) verbunden ist.

6. Gelenkprothese nach Anspruch 5, wobei die Adduktionsmittel (40) des Daumens (2) eine steife, im Wesentlichen U-förmige Halterung (41) umfassen, die mit der Grundphalanx (22) des Daumens (2) verbunden ist, wobei die Halterung (41) drehbar mit der Mittelhand (21) um die Adduktionsachse (73) verbunden ist und die Adduktionsachse (73) drehbar mit dem angetriebenen Element (72) des Malteserkreuz- oder Sternradmechanismus (70) verbunden ist und das Antriebsrad (71) des Mechanismus (70) drehbar mit einer Antriebsachse (5) verbunden ist, die im Wesentlichen parallel zur Adduktionsachse (73) verläuft.

7. Hand-Gelenkprothese nach Anspruch 1, wobei die Beugungsmittel (30) das um eine im Wesentlichen parallel zur Beugungsachse (4) verlaufende Antriebsachse (69) rotierende Hauptelement (12) umfassen sowie ein Kurbelstangenelement (11), aufweisend ein erstes Ende, das mittels eines Scharniers mit dem rotierenden Hauptelement (12) verbunden ist, und zwar an einer Stelle (68) außerhalb der Antriebsachse (69), sowie ein zweites Ende, das mittels eines Scharniers mit der Grundphalanx (23)des Zeigefingers verbunden ist, und zwar an einer Stelle (27) außerhalb der Beugungsachse (4).

8. Gelenkprothese nach Anspruch 3 und 7, wobei das Kurbelstangenelement (11) aus elastischen, weichen Materialien oder Kabeln oder deren Kombinationen besteht.

9. Gelenkprothese nach Anspruch 3 und 7, wobei die Übertragungsmittel (50) eine Leitung zur Übertragung der Bewegung (55) zwischen dem rotierenden Hauptelement (12) und dem Antriebsrad (71) des Mechanismus (70) umfassen.

10. Gelenkprothese nach Anspruch 3 und 7, wobei es sich beim rotierenden Hauptelement (12) und dem Antriebsrad (71) um Zahnradsegmente handelt, die eingreifen, oder um Zahnräder, die eingreifen.

11. Gelenkprothese nach einem der vorhergehenden Ansprüche, wobei es sich beim Elektromotor (24) um einen Rotationsmotor mit einer auf dessen Welle montierten Endlosschraube (7) handelt, wobei die Endlosschraube (7) mit einem Zahnrad für den Bewegungseinlauf (6) in Eingriff gelangt und das Einlaufrad (6) koaxial zum rotierenden Hauptelement (12) angeordnet und fest mit diesem verbunden ist.

12. Gelenkprothese nach einem der vorhergehenden Ansprüche, umfassend außer dem Daumen (2) und dem Zeigefinger (3) auch die übrigen drei Finger (33) der Hand, die mittels eines Scharniers mit den jeweiligen Beugungsachsen(34, 35) verbunden sind, betätigt über einen zweiten Elektrorotationsmotor, (25), der in die Prothese (20) integriert ist, wobei dieser Motor (25) eine Endlosschraube (28) umfasst, die auf dessen Welle montiert ist, gekuppelt mit einem weiteren Zahnrad (29), das mit einer zweiten Übertragungsleitung (27) verbunden ist und die Beugebewegung der weiteren drei Finger (33) um die Beugungsachsen (34, 35) betätigt.

## Revendications

1. Prothèse articulée de la main (20) ayant un métacarpe (21) qui se prolonge essentiellement le long d'un plan métacarpien (M-M), et au moins un pouce (2) ayant une phalange proximale (22) reliée de façon pivotante au métacarpe (21) et un index (3) ayant une phalange proximale (23) reliée de façon pivotante au métacarpe (21), comprenant:
- moyens de flexion (30) de l'index (3) qui agissent pour faire pivoter l'index (3) autour d'un axe de flexion (4) essentiellement parallèle au plan métacarpien (M-M);
- moyens d'adduction (40) du pouce (2) qui agissent pour faire tourner le pouce (2) autour d'un axe d'adduction (73) essentiellement parallèle au plan métacarpien (M-M), et incliné par rapport à l'axe de flexion de l'index (4);
- un moteur électrique (24) intégré dans la prothèse (20) ;
- les moyens de transmission du mouvement (50) entre le moteur électrique (24) et les moyens de flexion (30) et d'adduction (40), sont des moyens de transmission (50) de type mécanique;
- un élément rotatif principal (12) actionné par le moteur électrique (24);
- dans lequel les moyens de transmission (50) sont configurés afin de contrôler de manière sélective les moyens de flexion (30) et les moyens d'adduction (40);
Il se **caractérise par**:
les moyens de transmission (50) qui sont configurés de telle sorte qu'un premier mouvement angulaire (60) de l'élément rotatif principal (12) entre une première position angulaire (61) et une seconde position angulaire (62), produit une importante rotation de flexion (66) de la phalange proximale (23) de l'index (3) entre une position fermée et une position ouverte, tandis que les moyens d'adduction (40) du pouce (2) restent bloqués pendant le premier mouvement angulaire (60) de l'élément rotatif principal (12) entre la première position angulaire (61) et la deuxième position angulaire (62).

2. Prothèse articulée, selon la revendication 1, dans laquelle les moyens de transmission (50) sont configurés pour maintenir les moyens d'adduction bloqués (40) du pouce et commander simultanément l'ouverture et la fermeture lors des moyens de flexion (30) de l'index, en correspondance d'au moins un mouvement du moteur électrique (24) d'une première position à une seconde position.

3. Prothèse articulée, selon la revendication 1 ou 2, dans lesquelles les moyens de transmission (50) sont configurés pour transformer un mouvement de rotation continu d'un élément rotatif principal (12) actionné par le moteur (24), en un mouvement intermittent des moyens d'adduction (40).

4. Prothèse articulée, selon la revendication 3, dans laquelle les moyens de transmission (50) sont configurés de sorte que:
- au premier déplacement angulaire (60) de l'élément rotatif principal (12) entre la première position angulaire (61) et la deuxième position angulaire (62), correspond une rotation significative de flexion (66) de la phalange proximale (23) de l'index (3) et une position de blocage de la rotation des moyens d'adduction (40) du pouce (2);
- au deuxième mouvement angulaire (63) de l'élément rotatif principal (12) entre la seconde position angulaire (62) et une troisième position angulaire (64) correspond une position d'absence de rotation de la phalange proximale (23) de l'index (3) et une rotation évidente (87) des moyens d'adduction (40) du pouce (2); -
- au troisième mouvement angulaire (65) de l'élément rotatif principal (12) entre la troisième position angulaire (64) et une quatrième position angulaire (66) correspond une rotation de flexion (68) de la phalange proximale (23) de l'index (3) et une position de blocage de la rotation des moyens d'adduction (40) du pouce (2).

5. Prothèse articulée, selon la revendication 4, dans laquelle les moyens de transmission (50) comprennent un mécanisme de croix de Malte ou transversal de Genève (70), dans lequel la roue motrice (71) du mécanisme est reliée cinématiquement avec l'élément rotatif principal (12) et, la roue menée (72) du mécanisme fait partie intégrante des moyens d'adduction (40) du pouce (2).

6. Prothèse articulée selon la revendication 5 dans laquelle les moyens d'adduction (40) du pouce (2) comprennent un support rigide (41) essentiellement en forme caractéristique de U et relié à la phalange proximale (22) du pouce (2), ce support (41) étant relié de manière rotative au métacarpe (21) autour de l'axe d'adduction (73), cet axe d'adduction (73) étant relié de manière rotative à l'élément conducteur (72) du mécanisme de la croix de Malte ou transversale de Genève (70), la roue motrice (71) du mécanisme (70) étant reliée de manière rotative à un axe moteur (5) essentiellement parallèle à l'axe d'adduction (73).

7. Prothèse articulée de la main, selon la revendication 1, dans laquelle les moyens de flexion (30) comprennent l'élément principal (12) tournant autour d'un axe moteur (69) essentiellement parallèle à l'axe de flexion (4), et un élément de tige de liaison (11) ayant une première extrémité articulée sur l'axe principal de rotation (12) en un point (68) externe à l'axe moteur (69) et une deuxième extrémité articulée sur la phalange proximale (23) de l'index dans un point (27) à l'extérieur de l'axe de flexion (4).

8. Prothèse articulée, selon les revendications 3 et 7, dans lesquelles l'élément de connexion de la tige (11) est réalisé à partir de matériaux élastiques, de ressorts ou de câbles, ou leur combinaison d'assemblage.

9. Prothèse articulée, selon les revendications 3 et 7, dans lesquelles les moyens de transmission (50) comprennent une ligne de transmission de mouvement (55) entre l'élément rotatif principal (12) et la roue motrice (71) du mécanisme (70).

10. Prothèse articulée, selon les revendications 3 et 7, dans lesquelles le principal élément rotatif (12) et la roue motrice (71) sont des éléments d'engrenage de la roue dentée, soit des roues dentées.

11. Prothèse articulée, selon l'une des revendications précédentes, dans lesquelles le moteur électrique (24) est du type rotatif comportant une vis sans fin (7) montée sur son arbre, la vis sans fin (7) engrenant avec une roue dentée d'entrée du mouvement (6), dite roue d'entrée (6) étant coaxial et solidaire avec cet élément principal de rotation (12).

12. Prothèse articulée, selon l'une des revendications précédentes, qui comprend en plus du pouce (2) et de l'index (3), également les trois autres doigts (33) de la main articulées autour d'axes respectifs de flexion (34,35), actionnés par un second moteur électrique rotatif (25) intégré dans la prothèse (20), dit moteur (25) comprenant une vis sans fin (28) montée sur son arbre, couplée à une roue dentée supplémentaire (29) reliée à une seconde ligne de transmission (27) qui actionne le mouvement de flexion des autres trois doigts (33) autour des axes de flexion (34,35) .
